# EUROPEAN PATENT SPECIFICATION

(11) **EP 1 790 702 B1**
(45) Date of publication and mention of the grant of the patent: **18.02.2009**
(21) Application number: 06256015.6
(22) Date of filing: 24.11.2006
(51) Int. Cl.: C09D 133/04, C09D 131/04, C09D 133/14, A61L 31/10

(54) **Amphiphilic copolymer compositons**
Amphiphile Copolymerzusammensetzungen
Compositions copolymères amphiphiles

(30) Priority: 29.11.2005 US 288760
(43) Date of publication of application: 30.05.2007
(73) Proprietor: Cordis Corporation, Miami Lakes, FL 33014 (US)
(72) Inventor: Narayanan, Pallassana V, Belle Mead - NJ 08502 (US); Zhao, Jonathon Z, Belle Mead NJ-08502 (US)
(74) Representative: Belcher, Simon James

(56) References cited:
- US-A1- 4 562 226

## Description

The present invention relates to an amphiphilic coating material for application to at least a portion of one surface of an article. The present invention also relates to an article having the amphiphilic coating.

Most medical devices are made from metals, ceramics, or polymeric materials. However, these materials are hydrophobic, non-conformal, and non-slippery, and thereby may cause thrombus formation, inflammation, or other injuries to mucous membranes during use or operation. Thus, the issue of biocompatibility is a critical concern for manufacturers of medical devices, particularly medical implants. In order to function properly and safely, medical devices are usually coated with one or more layers of biocompatible materials. The coatings on these medical devices may, in some instances, be used to deliver therapeutic and pharmaceutical agents.

Since medical devices, particularly implantable medical devices, are intended for prolonged use and directly interface with body tissues, body fluids, electrolytes, proteins, enzymes, lipids, and other biological molecules, the coating materials for medical devices must meet stringent biological and physical requirements. These requirements, as a minimum, include the following: (1) the coatings must be hydrophilic and lubricous when in contact with body tissue, and thereby increase patient comfort during operation and enhance the manoeuvrability of the medical device; (2) the coatings must be flexible and elastic, so they conform to the biological structure without inducing detrimental stress; (3) the coatings must be haemocompatible, and thereby reduce or avoid formation of thrombus or emboli; (4) the coatings must be chemically inert to body tissue and body fluids; and (5) the coatings must be mechanically durable and not crack when formed on medical devices. If the coatings are impregnated with pharmaceutical or therapeutic agents, it is typically required that the coatings and the formation thereof are compatible with the pharmaceutical or therapeutic agents. If the coatings are used as coatings and the underlying basecoats are impregnated with pharmaceutical or therapeutic agents, it is further required that the coating and the formation thereof must be compatible with the basecoat and the pharmaceutical or therapeutic agents impregnated therein; and the coating must allow the pharmaceutical or therapeutic agents to permeate through it. It is also desirable that the coating functions as a physical barrier, a chemical barrier, or a combination thereof to control the elution of the pharmaceutical or therapeutic agents in the underlying basecoat.

In order to combine the desired properties of different polymeric materials, the conventional coating composition for commercial drug eluting stents used a polymer blend, i.e., physical mixture, of poly ethylene-vinyl acetate (EVAc) and poly butyl methacrylate (BMA). However, one disadvantage of this conventional coating is the phase separation of the polymer blend, which can be detrimental to the performance of the coating and the stability of drugs impregnated therein.

Another coating composition of the prior art comprises a supporting polymer and a hydrophilic polymer, wherein the supporting polymer contains functional moieties capable of undergoing crosslinking reactions and the hydrophilic polymer is associated with the supporting polymer (for example as disclosed in US-6238799). However, the preparation of this prior art coating composition employs chemical crosslinking reactions and a high temperature curing process, which are not compatible with a drug-containing coating.

The prior art also uses a coating composition formed by the gas phase or plasma polymerization of a gas comprising monomers of polyethylene glycol vinyl ether compounds (for example as disclosed in US-A-2003/0113477). However, the polymer prepared through the plasma process has poorly defined molecular weight and a large polydispersity. The plasma laid polymers of low molecular weight have limited mechanical durability. Further, plasma treatment can penetrate through the underlying basecoat and damage the drug content therein. Another problem with this prior art approach is that the free radicals or other high energy species generated in the plasma process may persist in the coating and cause drug content loss in the basecoat over time.

To decrease thrombosis caused by the use of medical devices, the prior art modifies the coatings of medical devices via conjugating, i.e., covalently bonding, an antithrombotic agent to the coatings (for example as disclosed in US-4973493 and at www.surmodics.com). Although this approach may produce a coating with excellent antithrombotic property, the prior art conjugation methods employ UV-radiating processes and/or chemical crosslinking processes, which may cause degradation of the antithrombotic agent in the coating.

Thus, there remains a need for a coating material that can satisfy the stringent requirements, as described above, for applying on at least one surface of a medical device and can be prepared through a process that is compatible with the sensitive pharmaceutical or therapeutic agents impregnated in the coatings.

US-4562226 discloses aqueous compositions suitable for forming a temporary protective coating on a substrate. The temporary protective coating can subsequently be removed by treatment with an aqueous alkaline solution. The composition comprises a sterically stabilised dispersion in an aqueous medium of particles of size range 0.1-10 µm of a polymer derived from monomers including at least one acrylic monomer and from 5% to 40%, based on the total weight of monomers, of an ethylenically unsaturated monocarboxylic acid, or an equivalent proportion of an unsaturated polycarboxylic acid.

Accordingly, the present invention provides an amphiphilic coating material for applying on at least a portion of one surface of an article, characterised in that the article is a medical device or a component of a medical device. By "amphiphilic", it is meant having the property of hydrophobicity and hydrophilicity simultaneously. The amphiphilic coating material comprises a copolymer containing one or more alkyl methacrylate or alkyl acrylate co-monomer units; one or more vinyl acetate co-monomer units; and up to 40% mole of polyethylene oxide substituted methacrylate co-monomer units. Optionally, one or more biologically active molecules may be covalently bonded to the polyethylene oxide substituted methacrylate co-monomer units.

Preferably, the polyethylene oxide substituted methacrylate co-monomer unit comprises the following structure: 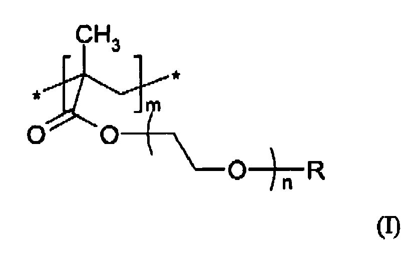 wherein R is a hydrogen atom, an alkyl group of 1 to 6 carbon atoms, or a biologically active molecule; n is an integer of 2 to 100; and m is an integer of 100 to 5000.

The present invention also provides an article having an amphiphilic coating thereon. The amphiphilic coating comprises a copolymer containing one or more alkyl methacrylate or alkyl acrylate co-monomer units; one or more vinyl acetate co-monomer units; and up to 40% mole of polyethylene oxide substituted methacrylate co-monomer units. Optionally, one or more biologically active molecules may be covalently bonded to the polyethylene oxide substituted methacrylate co-monomer units. The article is a medical device or a component of a medical device.

The present invention provides an amphiphilic coating material for applying on at least a portion of one surface of an article. The amphiphilic coating material comprises a copolymer containing one or more alkyl methacrylate or alkyl acrylate co-monomer units; one or more vinyl acetate co-monomer units; and up to 40% mole of polyethylene oxide substituted methacrylate co-monomer unites. The co-monomers in the copolymer, i.e., the alkyl methacrylate or alkyl acrylate co-monomers, the vinyl acetate co-monomers, and the polyethylene oxide substituted methacrylate co-monomers, are in random sequence. By "alkyl methacrylate", it is meant a methacrylate derivative wherein the oxygen atom attached to the carbon atom of the carbonyl group is substituted with an alkyl group. By "alkyl acrylate", it is meant an acrylate derivative wherein the oxygen atom attached to the carbon atom of the carbonyl group is substituted with an alkyl group. By "polyethylene oxide substituted methacrylate", it is meant a methacrylate derivative wherein the oxygen atom attached to the carbon atom of the carbonyl group is substituted with a polyethylene oxide moiety.

The copolymer has a hydrophobic backbone that is formed by polymerization of vinyl groups. The polyethylene oxide moieties of the polyethylene oxide substituted methacrylate co-monomers provide hydrophilic pendent chains that are interspersed along the hydrophobic backbone. The polyethylene oxide moieties of the polyethylene oxide substituted methacrylate co-monomers also provide functional groups where one or more biologically active molecules may be attached. The "biologically active molecule" as used herein denotes a compound or substance having an effect on or eliciting a response from living tissue. The biologically active molecule is attached to the polyethylene oxide moiety via forming a covalent bond with the oxygen atom at the far end position of the polyethylene oxide moiety. By "the oxygen atom at the far end position", it is meant the oxygen atom of the polyethylene oxide moiety that is furthest apart from the carbonyl group in the polyethylene oxide substituted methacrylate. The hydrophilic pendent chains of the copolymer swell under the physiological condition and form a flexible and lubricious three-dimensional network, thereby providing a hydrophilic environment for retaining the optimal activity of the attached biologically active molecules.

Preferably, the polyethylene oxide substituted methacrylate co-monomer unit comprises the following structure: 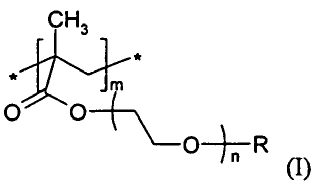 in which R is a hydrogen atom, an alkyl group having 1 to 6 carbon atoms, or a biologically active molecule; n is an integer of 2 to 100; and m is an integer of 100 to 5000. Preferably, n is an integer of 2 to 10. The alkyl group suitable for the present invention may be straight, branched, or cyclic. Examples of suitable alkyl groups include, but are not limited to: methyl, ethyl, propyl, isopropyl, butyl, isobutyl, *tert*-butyl, *n*-pentyl, cyclopropyl, cyclobutyl, and cyclopentyl. Preferably, the alkyl group is methyl.

The biologically active molecules are covalently bonded to the polyethylene oxide moieties through conjugation processes. The conjugation process may involve one or more chemical or photo radiation reactions. The biologically active molecules suitable for the present invention include, for example, any drugs, agents, compounds and/or combination thereof that have therapeutic effects for treating or preventing a disease or a biological organism's reaction to the introduction of the medical device to the organism. Preferred biological active molecules include, but are not limited to: anti-thrombogenic agents, immuno-suppressants, anti-neoplastic agents, anti-inflammatory agents, angiogenesis inhibitors, protein kinase inhibitors, and other agents which may cure, reduce, or prevent restenosis in a mammal. Examples of the biological active molecules of the present invention include, but are not limited to: heparin, albumin, streptokinase, tissue plasminogin activator (TPA), urokinase, rapamycin, paclitaxel, pimecrolimus, and their analogues and derivatives. When the copolymer comprises more than one biologically active molecules, the biologically active molecules can be the same or different.

Preferably, the alkyl methacrylate co-monomer unit has the following general formula: 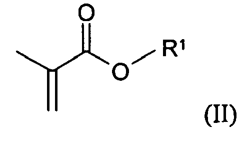 in which R¹ is an alkyl group having 1 to 12 carbon atoms. The alkyl group may be straight, branched, or cyclic. Examples of suitable alkyl groups include, but are not limited to: methyl, ethyl, propyl, isopropyl, butyl, isobutyl, *tert*-butyl, *n*-pentyl, cyclopropyl, cyclobutyl, and cyclopentyl. Preferably, the alkyl group is methyl or butyl.

Preferably, the alkyl acrylate co-monomer unit has the following general formula: 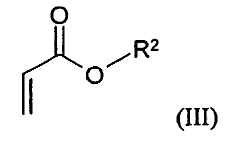 in which R² is an alkyl group having 1 to 12 carbon atoms. The alkyl group may be straight, branched, or cyclic. Examples of suitable alkyl groups include, but are not limited to: methyl, ethyl, propyl, isopropyl, butyl, isobutyl, tert-butyl, n-pentyl, cyclopropyl, cyclobutyl, and cyclopentyl. Preferably, the alkyl group is methyl or butyl.

When the copolymer comprises more than one alkyl methacrylate or alkyl acrylate co-monomers, the more than one methacrylate or acrylate co-monomers can be the same or different. Preferred alkyl methacrylate co-monomers include methyl methacrylate, ethyl methacrylate, butyl methacrylate, and cyclic alkyl methacrylate. Preferred alkyl acrylate co-monomers include methyl acrylate, ethyl acrylate, butyl acrylate, and cyclic alkyl acrylate. It is understood to one skilled in the art that suitable alkyl methacrylate or acrylate co-monomers also include any analogous alkyl methacrylates or alkyl acrylates of the above-mentioned alkyl methacrylate and alkyl acrylate co-monomers.

Preferably, a vinyl acetate monomer has the following general formula: 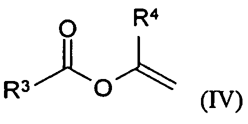 in which R³ is an alkyl group having 1 to 6 carbon atoms; and R⁴ is a hydrogen atom or an alkyl group having 1 to 6 carbon atoms. The alkyl group suitable for the present invention may be straight, branched, or cyclic. Preferably, the vinyl acetate co-monomer is a compound having the structure of formula (IV) wherein R³ is methyl and R⁴ is hydrogen. It is understood to one skilled in the art that suitable vinyl acetate co-monomers also include any analogous vinyl acetates of the above-mentioned vinyl acetate co-monomers.

The copolymer may comprise the following repeating unit: 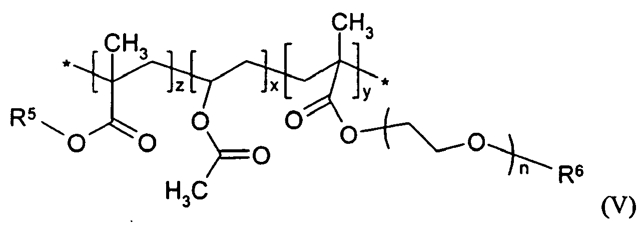 in which R⁵ is an alkyl group having 1 to 12 carbon atoms; R⁶ is a hydrogen atom or an alkyl group having 1 to 6 carbon atoms; n is an integer of 2 to 100; and x, y and z are the same or different, and are independently an integer of 10 to 2500. Preferably, n is an integer of 2 to 10.

The copolymer may comprise the following repeating unit: 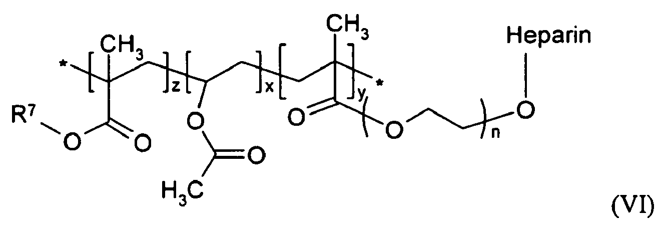 in which R⁷ is an alkyl group of 1 to 12 carbons; n is an integer of 2 to 100; and x, y and z are the same of different, and are independently an integer of 10 to 2500. Preferably, n is an integer of 2 to 10. Heparin is a well-known anticoagulant used to decrease the clotting ability of the blood and prevent harmful clots from forming in the blood vessels. The attachment of heparin to the polyethylene oxide moiety enables the resulting copolymer to be soluble in common organic solvents used in coating processes, thereby eliminating the use of water and improving the coating's morphology. A coating comprising the copolymer of formula (VI) has enhanced haemocompatibility, thus is particularly useful as the coating for implantable medical devices.

It is preferable that the copolymer has a tunable polymer molecular weight ranging from about 10 kDa to about 5000 kDa to enable the formation of a polymer with desirable mechanical durability and adequate adhesiveness. Since the mechanical durability of a coating improves upon increasing polymer molecular weight, it is especially preferable that the copolymer has a high polymer molecular weight of 50 kDa to 5000 kDa for use in coatings for certain medical devices (e.g., stents) which require expansion and deployment in *vivo.*

The amphiphilic coating of the invention may additionally include co-solvents and/or other additives to facilitate high quality film formation, such as plasticizers, antifoaming agents, anticrater agents, and coalescing solvents. Other suitable additives to the amphiphilic coating material include, but are not limited to: bioactive agents, antimicrobial agents, antithrombogenic agents, antibiotics, pigments, radiopacifiers and ion conductors. Details concerning the selection and amounts of such ingredients are known to those skilled in the art.

The amphiphilic coating material may be applied to at least a portion of one surface of an article. The coating may be applied to all exposed surfaces of an article. The thickness of the amphiphilic coating may vary depending on the process used in forming the coating as well as the intended use of the article. Typically, and for a medical device, the coating is applied to a thickness from about 1 to about 500 nm, with a thickness from about 2 to about 100 nm being more typical.

When applied on at least a portion of one surface of an article, the hydrophobic backbone of copolymer forms a non-swellable base layer and adheres firmly to the underlying surface, while the hydrophilic pendent chains of the copolymer hydrate and swell under physiological conditions and form a lubricious and haemocompatible surface. The low-friction and haemocompatibility of the hydrophilic pendent chains provide excellent anti-thrombotic properties that potentially reduce subacute thrombosis (SAT). Further, the hydrophobic backbone has a predefined molecular weight with a narrow range of distribution which improves the mechanical durability of the polymer, while the hydrophilic pendent chains are adjustable to various lengths to obtain the desirable elasticity of the polymer. Thus, the coating is robust, i.e., mechanically durable, and flexible, i.e., elastic. The robustness and flexibility of the polymer of the invention significantly reduce flaking, peeling, and other defects commonly seen in many current coatings on medical devices, particularly the coatings on stents. Accordingly, the present invention provides an improved biocompatible coating, which has not only inert hydrophilic surfaces to be in contact with body tissue of a mammal, for example, a human, sufficiently lubricious to reduce restenosis, or thrombosis, or other undesirable reactions, but also a hydrophobic backbone to firmly adhere to the underlying surface sufficiently durable to resist cracking when formed on an article, for example, a medical device.

The amphiphilic coating may also be applied to control the elution of a therapeutic dosage of a pharmaceutical agent from a medical device base coating, for example, a stent base coating. The basecoat generally comprises a matrix of one or more drugs, agents, and/or compounds and a biocompatible material such as a polymer. The control over elution results from either a physical barrier, or a chemical barrier, or a combination thereof. The elution is controlled by varying the thickness of the coating, thereby changing the diffusion path length for the drugs, agents, and/or compounds to diffuse out of the basecoat matrix. Essentially, the drugs, agents and/or compounds in the basecoat matrix diffuse through the interstitial spaces in the coating. Accordingly, the thicker the coating, the longer the diffusion path, and conversely, the thinner the coating, the shorter the diffusion path. It is important to note that both the basecoat and the coating thickness may be limited by the desired overall profile of the article on which they are applied.

The properties of the copolymer may be tuned via adjusting the molar ratios of the co-monomers. In other words, the molar ratios of the co-monomers may be adjusted according to the desired properties of the copolymer. For example, when biologically active molecules are attached to the polyethylene oxide substituted methacrylates, the co-monomers are in a molar ratio that ensures desired mechanical strength of the copolymer while providing a hydrophilic environment for retaining the optimal activity of the biologically active molecules. Preferably, the copolymer has the alkyl methacrylate or alkyl acrylate, the vinyl acetate, and the polyethylene oxide substituted methacrylate in a mole ratio of 1:1:1.

The structure of the hydrophobic backbone and the molecular weight of the polymer may be controlled through employment of various polymerization methods. The preferred polymerization methods of the present invention include group transfer polymerization (GTP), anionic polymerization, and living polymerization. The more preferred polymerization method of the present invention is GTP. GTP is a living polymerization technique which involves a Michael-type addition using a silyl ketene acetal initiator (see, for example, Vamvakaki, M. et al., Polymer, 40, 1999, 5161-5171). Many conventional polymerization methods require chemical crosslinking reactions, high temperature curing processes, and/or plasma treatments, which not only have very limited control over the polymer backbone structure and the molecular weight distribution, but also cause damage to the drug-content in the underlying basecoat. Unlike those conventional polymerization methods, GTP can be used for the synthesis of controlled structure acrylate or methacrylate polymers of narrow molecular weight distribution at ambient temperature. The hydrophilic pendent chains of the copolymer provide desired lubricious properties and haemocompatibility, and the length of these hydrophilic pendent chains can be controlled via using monomers with desirable number of repeating ethylene oxide units in the polymerization reactions. The preferred monomers for the polymerization are the monomers that contain 2 to 10 repeating ethylene oxide units. Moreover, the polyethylene oxide moieties serve as functional pendant chains whereby one or more biologically active molecules may be introduced via conjugating processes that are compatible with the biologically active molecules.

A general co-polymerization process is shown in Scheme 1 as below: 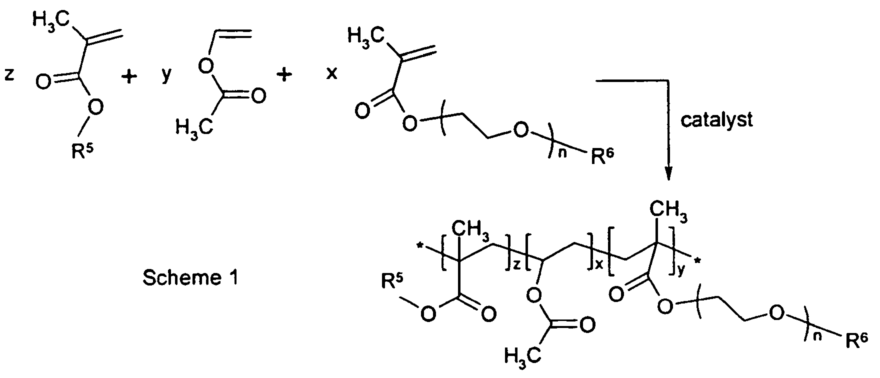 in which R⁵ is an alkyl group having 1 to 12 carbon atoms; R⁶ is a hydrogen atom or an alkyl group having 1 to 6 carbon atoms, x, y, and z are independently an integer of 10 to 2500, and n is an integer of 2 to 100. Catalysts suitable for the above polymerization process are known to one skilled in the art. Examples of the catalysts include, but are not limited to: 1-methoxy-1-trimethylsiloxy-2-methyl-1-propene (MTS), *n-*tetrabutylammonium bibenzoate (TBABB), and other polymerization initiators.

A general conjugation process is shown in Scheme 2 as below: 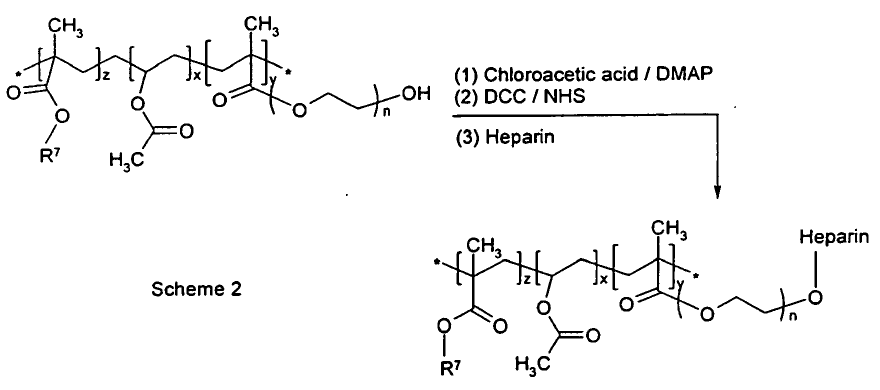 in which R⁷ is an alkyl group having 1 to 12 carbon atoms, x, y, and z are independently an integer of 10 to 2500, and n is an integer of 2 to 100. In the conjugation process illustrated by Scheme 2, the polyethylene oxide moiety is activated by treating the hydroxyl group with chloroacetic acid in the presence of DMAP (dimethylaminopyridine) and then DCC (dicyclohexylcarbodiimide) and NHS (N-hydroxyl succinimide). Next, heparin is conjugated with the activated polyethylene oxide moiety. The stepwise activation of the copolymer rather than derivatizing heparin not only minimizes the undesirable crosslinking reactions commonly seen in the derivatization of heparin, but also provides a high degree of control over conjugation extent. Further, the conjugation process of Scheme 2 is conducted under mild conditions, thereby eliminating the undesirable long-lasting free radicals generated in conventional UV-radiating conjugation process.

The present invention provides an article having the amphiphilic coating thereon. The amphiphilic coating is on at least a portion of one surface of the article. The at least a portion of one surface of the article may be a surface of a polymeric coat, a plastic substance, ceramic, steel, or other alloy metals. The article that is coated with the amphiphilic coating material may be in any shape, and is a medical device or a component of a medical device. The term "medical device" as used herein denotes a physical item used in medical treatment, which includes both external medical devices and implantable medical devices. The medical devices that are coated with the amphiphilic coating material include, but are not limited to: catheters, guidewires, drug eluting stents, cochlear implants, retinal implants, gastric bands, neurostimulation devices, muscular stimulation devices, implantable drug delivery devices, intraocular devices, and various other medical devices.

The amphiphilic coating material may be applied to the surface of an article using conventional coating techniques, such as, for example, spray coating, ultrasonic coating, dip coating, and the like. In a dip coating process, the article is immersed in a bath containing the amphiphilic coating material and then removed. A dwelling time ranging from about 1 minute to about 2 hours may be used depending of the material of construction, complexity of the device, and the desired coating thickness. Next, the article coated with the amphiphilic coating material may be allowed to dry to provide a dry coating. Drying may be accomplished merely by standing at ambient conditions or may be accelerated by heating at mild temperatures, such as about 30°C to about 65°C.

## Claims

1. An article having an amphiphilic coating thereon, said amphiphilic coating comprising a copolymer containing one or more alkyl methacrylate or alkyl acrylate co-monomer units; one or more vinyl acetate co-monomer units; and up to 40% mole of polyethylene oxide substituted methacrylate co-monomer units; **characterised in that** the article is a medical device or a component of a medical device.

2. The article of claim 1, wherein the polyethylene oxide substituted methacrylate co-monomer unit comprising the following structure: 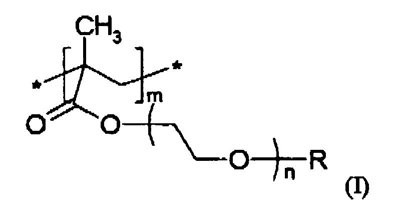 in which R is a hydrogen atom, an alkyl group of 1 to 6 carbon atoms, or a biologically active molecule; n is an integer of 2 to 100; and m is an integer of 10 to 500.

3. The article of claim 2, wherein n is an integer of 2 to 10.

4. The article of claim 2, wherein the biologically active molecule is an anti-thrombogenic agent, an immuno-suppressant agent, an anti-neoplastic agent, an antiinflammatory agent, an angiogenesis inhibitor, or a protein kinase inhibitor.

5. The article of claim 2, wherein the biologically active molecule is selected from the group consisting of heparin, rapamycin, paclitaxel, pimecrolimus, and the analogues and derivatives thereof.

6. The article of claim 1, wherein one or more biologically active molecules are covalently bonded to the polyethylene oxide substituted methacrylate co-monomer units.

7. The amphiphilic coating material of claim 1, wherein the one or more methacrylate co-monomers are selected from the group consisting of methyl methacrylate, ethyl methacrylate, butyl methacrylate, hexyl methacrylate, and octyl methacrylate.

8. The amphiphilic coating material of claim 1, wherein the one or more acrylate co-monomers are selected from the group consisting of methyl acrylate, ethyl acrylate, butyl acrylate, hexyl acrylate, and octyl acrylate.

9. The article of claim 1, wherein the amphiphilic coating has a thickness of about 1 to about 500 nm.

10. The article of claim 1, wherein the copolymer has a tunable molecular weight ranging from about 10 kDa to about 500 kDa.

11. The article of claim 1, wherein the copolymer comprises the following repeating unit: 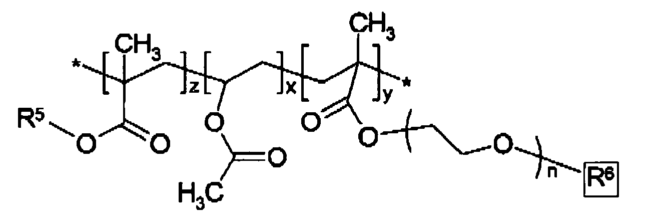 in which R⁵ is an alkyl group having 1 to 12 carbon atoms; R⁶ is a hydrogen atom or an alkyl group having 1 to 6 carbon atoms; n is an integer of 2 to 100; and x, y and z are the same or different, and are independently an integer of 10 to 5000.

12. The article of claim 1, further comprising one or more biologically active molecules covalently bonded to the polyethylene oxide substituted methacrylate co-monomer units.

13. The article of claim 12, wherein the copolymer comprises the following repeating unit: 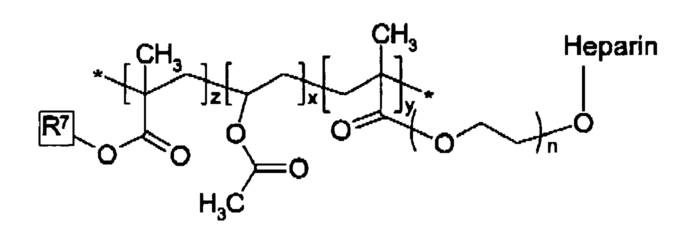 in which R⁷ is a hydrogen atom or an alkyl group of 1 to 6 carbons; n is an integer of 2 to 100; and x, y and z are the same or different, and are independently an integer of 10 to 2500.

14. An amphiphilic coating material for applying to the article of claim 1, wherein the copolymer comprises the following repeating unit: 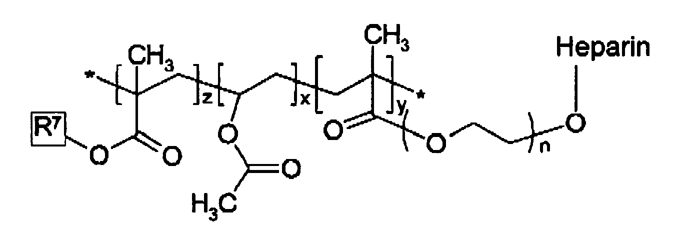 in which R⁷ is an alkyl group of 1 to 12 carbons; n is an integer of 2 to 100; and x, y and z are the same or different, and are independently an integer of 10 to 2500.

## Patentansprüche

1. Artikel mit einer amphiphilen Beschichtung darauf, wobei die amphiphile Beschichtung ein Copolymer, enthaltend ein oder mehrere Alkylmethacrylat- oder Alkylacrylat-Comonomer-Einheiten; ein oder mehrere Vinylacetat-Comonomer-Einheiten und bis zu 40 Mol% an Polyethylenoxid-substituierten Methacrylat-Comonomer-Einheiten umfasst, **dadurch gekennzeichnet, dass** der Artikel eine medizinische Vorrichtung oder ein Bestandteil einer medizinischen Vorrichtung ist.

2. Artikel nach Anspruch 1, wobei die Polyethylenoxid-substituierte Methacrylat-Comonomer-Einheit die folgende Struktur umfasst: 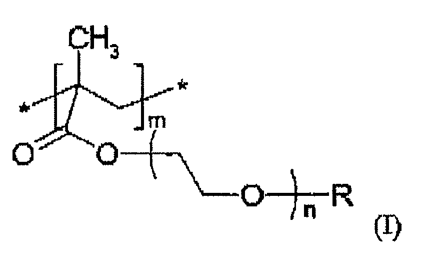 wobei R ein Wasserstoffatom, eine Alkylgruppe mit 1 bis 6 Kohlenstoffatomen, oder ein biologisch aktives Molekül ist, n eine ganze Zahl von 2 bis 100 ist; und m eine ganze Zahl von 10 bis 500 ist.

3. Artikel nach Anspruch 2, wobei n eine ganze Zahl von 2 bis 10 ist.

4. Artikel nach Anspruch 2, wobei das biologisch aktive Molekül ein anti-thrombogenes Agens, ein immun-supprimierendes Agens, ein anti-neoplastisches Agens, ein antientzündliches Agens, ein Angiogenese-Inhibitor oder ein Proteinkinase-Inhibitor ist.

5. Artikel nach Anspruch 2, wobei das biologisch aktive Molekül ausgewählt ist aus der Gruppe, bestehend aus Heparin, Rapamycin, Paclitaxel, Pimecrolimus und die Analoge und Derivate davon.

6. Artikel nach Anspruch 1, wobei ein oder mehrere biologisch aktive Moleküle an die Polyethylenoxid-substituierten Methacrylat-Comonomer-Einheiten kovalent gebunden sind.

7. Amphiphiles Beschichtungsmaterial nach Anspruch 1, wobei das eine oder die mehreren Methacrylat-Comonomere ausgewählt sind aus der Gruppe, bestehend aus Methylmethacrylat, Ethylmethacrylat, Butylmethacrylat, Hexylmethacrylat und Octylmethacrylat.

8. Amphiphiles Beschichtungsmaterial nach Anspruch 1, wobei das eine oder die mehreren Acrylat-Comonomere ausgewählt sind aus der Gruppe, bestehend aus Methylacrylat, Ethylacrylat, Butylacrylat, Hexylacrylat und Octylacrylat.

9. Artikel nach Anspruch 1, wobei die amphiphile Beschichtung eine Dicke von ungefähr 1 bis ungefähr 500 nm hat.

10. Artikel nach Anspruch 1, wobei das Copolymer ein einstellbares Molekulargewicht im Bereich von ungefähr 10 kDa bis ungefähr 500 kDa hat.

11. Artikel nach Anspruch 1, wobei das Copolymer die folgende Wiederholungseinheit umfasst: 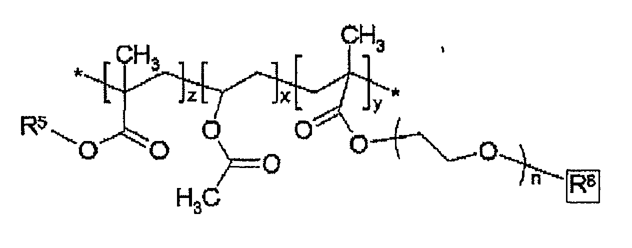 wobei R⁵ eine Alkylgruppe mit 1 bis 12 Kohlenstoffatomen ist; R⁶ ein Wasserstoffatom oder eine Alkylgruppe mit 1 bis 6 Kohlenstoffatomen ist; n eine ganze Zahl von 2 bis 100 ist; und x, y und z dieselben oder unterschiedlich sind und unabhängig eine ganze Zahl von 10 bis 5000 sind.

12. Artikel nach Anspruch 1, weiterhin umfassend ein oder mehrere biologisch aktiven Moleküle, die kovalent an die Polyethylenoxid-substituierten Methacrylat-Comonomer-Einheiten gebunden sind.

13. Artikel nach Anspruch 12, wobei das Copolymer die folgende Wiederholungseinheit umfasst: 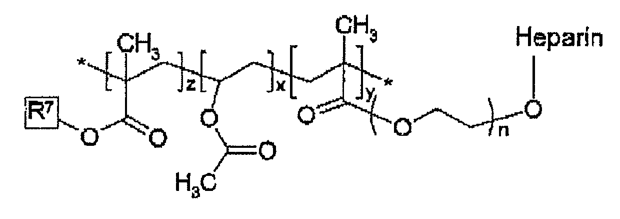 wobei R⁷ ein Wasserstoffatom oder eine Alkylgruppe mit 1 bis 6 Kohlenstoffen ist; n eine ganze Zahl von 2 bis 100 ist; und x, y und z dieselben oder unterschiedlich sind und unabhängig eine ganze Zahl von 10 bis 2500 sind.

14. Amphiphiles Beschichtungsmaterial zur Auftragung auf den Artikel nach Anspruch 1, wobei das Copolymer die folgende Wiederholungseinheit umfasst: 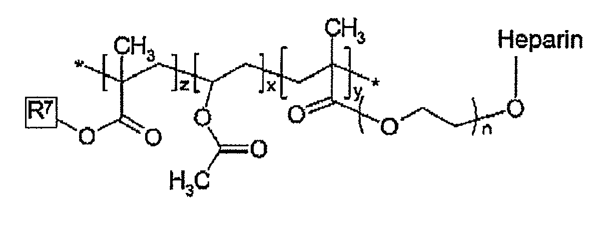 wobei R⁷ eine Alkylgruppe mit 1 bis 12 Kohlenstoffen ist; n eine ganze Zahl von 2 bis 100 ist; und x, y und z dieselben oder unterschiedlich sind und unabhängig eine ganze Zahl von 10 bis 2500 sind.

## Revendications

1. Article ayant un revêtement amphiphile sur celui-ci, ledit revêtement amphiphile comprenant un copolymère contenant un ou plusieurs motifs comonomères de méthacrylate d'alkyle ou d'acrylate d'alkyle ; un ou plusieurs motifs comonomères d'acétate de vinyle ; et jusqu'à 40 % en moles de motifs comonomères de méthacrylate substitué par un oxyde de polyéthylène; **caractérisé en ce que** l'article est un dispositif médical ou un composant d'un dispositif médical.

2. Article selon la revendication 1, dans lequel le motif comonomère de méthacrylate substitué par un oxyde de polyéthylène comprend la structure suivants : 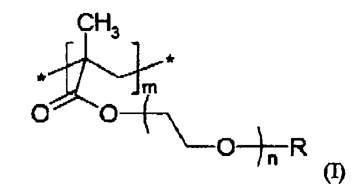 dans laquelle R est un atome d'hydrogène, un groupe alkyle de 1 à 6 atomes de carbone, ou une molécule biologiquement active ; n est un nombre entier de 2 à 100 ; et m est un nombre entier de 10 à 500.

3. Article selon la revendication 2, dans lequel n est un nombre entier de 2 à 10.

4. Article selon la revendication 2, dans lequel la molécule biologiquement active est un agent anti-thrombogène, un agent immunosuppresseur, un agent anti-néoplasique, un agent anti-inflammatoire, un inhibiteur de l'angiogenèse, ou un inhibiteur de la protéine kinase.

5. Article selon la revendication 2, dans lequel la molécule biologiquement active est choisie dans le groupe constitué par l'héparine, la rapamycine, le paclitaxel, le pimécrolimus, et leurs analogues et dérivés.

6. Article selon la revendication 1, dans lequel une ou plusieurs molécules biologiquement actives sont liées de manière covalente aux motifs comonomères de méthacrylate substitué par un oxyde de polyéthylène.

7. Matériau de revêtement amphiphile selon la revendication 1, dans lequel le ou les comonomères de méthacrylate sont choisis dans le groupe constitué par le méthacrylate de méthyle, le méthacrylate d'éthyle, le méthacrylate de butyle, le méthacrylate d'hexyle et le méthacrylate d'octyle.

8. Matériau de revêtement amphiphile selon la revendication 1, dans lequel le ou les comonomères d'acrylate sont choisis dans le groupe constitué par l'acrylate de méthyle, l'acrylate d'éthyle, l'acrylate de butyle, l'acrylate d'hexyle, et l'acrylate d'octyle.

9. Article selon la revendication 1, dans lequel le composé amphiphile a une épaisseur d'environ 1 à environ 500 nm.

10. Article selon la revendication 1, dans lequel le copolymère a un poids moléculaire ajustable, variant entre environ 10 kDa à environ 500 kDa.

11. Article selon la revendication 1, dans lequel le copolymère comprend le motif répétitif suivant : 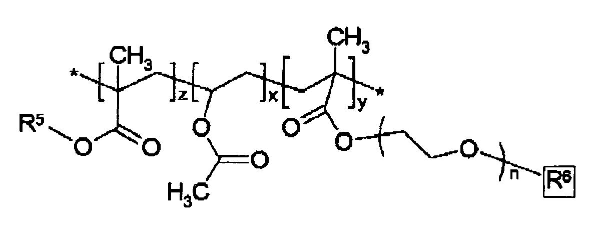 dans lequel R⁵ est un groupe alkyle ayant de 1 à 12 atomes de carbone ; R⁶ est un atome d'hydrogène ou un groupe alkyle ayant de 1 à 6 atomes de carbone ; n est un nombre entier de 2 à 100 ; et x, y et z sont identiques ou différents, et sont indépendamment un nombre entier de 10 à 5000.

12. Article selon la revendication 1, comprenant en outre une ou plusieurs molécules biologiquement actives liées de manière covalente aux motifs comonomères de méthacrylate substitué par un oxyde de polyéthylène.

13. Article selon la revendication 12, dans lequel le copolymère comprend le motif répétitif suivant : 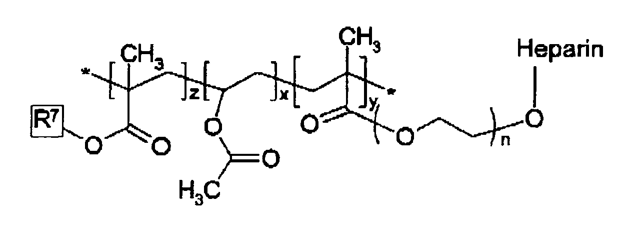 dans lequel R⁷ est un atome d'hydrogène ou un groupe alkyle ayant de 1 à 6 atomes de carbone ; n est un nombre entier de 2 à 100 ; et x, y et z sont identiques ou différents, et sont indépendamment un nombre entier de 10 à 2500.

14. Matériau de revêtement amphiphile pour une application sur l'article selon la revendication 1, dans lequel le copolymère comprend le motif répétitif suivant : 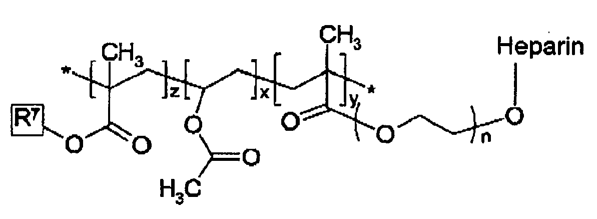 dans lequel R⁷ est un groupe alkyle constitué de 1 à 12 atomes de carbone ; n est un nombre entier de 2 à 100 ; et x, y et z sont identiques ou différents, et sont indépendamment un nombre entier de 10 à 2500.
